# EUROPÄISCHE PATENTANMELDUNG

(11) **EP 3 492 141 A1**
(43) Veröffentlichungstag der Anmeldung: **05.06.2019**
(21) Anmeldenummer: 17204670.8
(22) Anmeldetag: 30.11.2017
(51) Int. Cl.: A61N 1/372, H01Q 1/22, H01Q 1/27, H01Q 1/36, H01Q 9/42, A61N 1/375

(54) **IMPLANTIERBARES MEDIZINELEKTRONISCHES GERÄT UND SENDE-/EMPFANGSANTENNE FÜR EIN SOLCHES**

(71) Anmelder: BIOTRONIK SE & Co. KG, 12359 Berlin (DE)
(72) Erfinder: Hartmann-Bax, Kathy, 14947 Nuthe-Urstromtal (DE); Ruschel, Marina, 12679 Berlin (DE)
(74) Vertreter: Keck, Hans-Georg

(57) **Zusammenfassung**

Implantierbares medizinelektronisches Gerät, welches einen Gerätekörper und einen hierauf aufgesetzten Header aufweist, mit einer Telemetrie-Baugruppe zur Signalübertragung nach und/oder von außerhalb des Körpers eines Patienten im implantierten Zustand des Gerätes, insbesondere zur drahtlosen bidirektionalen Kommunikation, wobei der Telemetrie-Baugruppe mindestens größtenteils eine aus einem langgestreckten Leiter räumlich geformte, im Bereich des Headers angeordnete Sende-/Empfangsantenne zugeordnet ist, welche derart konfiguriert ist, dass sie einen Formschluss mit der Außenkontur von mindestens einem Teil des Headers hat und hierdurch im Header fixiert ist.

## Beschreibung

Die Erfindung betrifft ein implantierbares medizinelektronisches Gerät, welches einen Gerätekörper und einen hierauf aufgesetzten Header aufweist, mit einer Telemetrie-Baugruppe zur Signalübertragung nach und/oder von außerhalb des Körpers eines Patienten im implantierten Zustand des Gerätes, insbesondere zur drahtlosen bidirektionalen Kommunikation, wobei der Telemetrie-Baugruppe mindestens größtenteils eine aus einem langgestreckten Leiter räumlich geformte, im Bereich des Headers angeordnete Sende-/Empfangsantenne zugeordnet ist. Sie betrifft des Weiteren eine entsprechende Sende-/Empfangsantenne.

Es ist seit langem Praxis, dass implantierbare medizinelektronische Geräte über kurzreichweitige drahtlose Nachrichtenverbindungen, die sogenannte Telemetrie, im Körper aufgenommene Messwerte oder Zustandswerte des Gerätes nach außerhalb des Körpers des Patienten übermitteln bzw. von außen Steuer- bzw. Programmiersignale empfangen. Wie für jede Funkverbindung, sind für diese Kommunikation Antennen erforderlich. Die im implantierbaren Gerät vorgesehene Antenne sitzt außerhalb des elektromagnetisch abschirmenden Gerätegehäuses, also bei Geräten mit einem sogenannten Header (Kopfteil) im Bereich dieses Headers.

Wie alle Komponenten implantierbarer medizinelektronischer Geräte, sind auch die Telemetrie-Antennen Gegenstand ständiger Weiterentwicklung. Beispiele moderner Antennen dieser Art sind etwa in der US 7,317,946 B2 oder der EP 2 643 050 B1 beschrieben. Beide Druckschriften zeigen die Ausführung solcher Antennen mit einer Spiral- bzw. Mäanderstruktur zur Vergrößerung der wirksamen Leiterlänge unter gleichzeitiger Wahrung der erforderlichen Kompaktheit. Die US 7,317,946 B2 beschreibt im Übrigen, wie eine Sende-/Empfangsantenne der gattungsgemäßen Art platzsparend im Header eines implantierbaren Gerätes untergebracht werden und zugleich eine vorteilhafte Antennencharakteristik realisiert werden kann.

Der Erfindung liegt die Aufgabe zugrunde, ein weiter verbessertes implantierbares medizinelektronisches Gerät und insbesondere eine Sende-/Empfangsantenne für die Telemetriefunktion eines solchen Geräts anzugeben, wobei insbesondere ein geringer Montageaufwand und entsprechend niedrige Montagekosten mit vorteilhaften elektromagnetischen Eigenschaften kombiniert sein sollen.

Diese Aufgabe wird durch ein Gerät mit den Merkmalen des Anspruchs 1 und speziell durch eine Sende-/Empfangsantenne mit den Merkmalen des Anspruchs 11 gelöst. Zweckmäßige Fortbildungen des Erfindungsgedankens sind Gegenstand der abhängigen Ansprüche.

Erfindungsgemäß ist die Sende-/Empfangsantenne derart konfiguriert, dass sie einen Formschluss mit der Außenkontur von mindestens einem Teil des Headers hat und hierdurch auf dem Header fixiert ist. Es versteht sich, dass es neben der hier gemeinten Fixierung noch eine Verbindung eines Antennenendes mit einem Anschlussstift im Header gibt, die etwa als Schweiß-, Löt-, Leitklebeverbindungen oder alternativ auch als Crimp-, Steck- oder Klemmverbindung ausgeführt sein kann, die jedoch primär der elektrischen Verbindung zwischen der Telemetrie-Baugruppe und der Antenne dient.

In einer vorteilhaften Ausgestaltung ist wenigstens ein Abschnitt des räumlich geformten langgestreckten Leiters federelastisch ausgeführt, derart, dass die Sende-/Empfangsantenne durch elastischen Formschluss auf dem Header fixiert ist. Diese Ausgestaltung ermöglicht eine besonders einfache Montage durch "Aufklipsen" der Antenne auf den ansonsten vormontierten Innenaufbau des Headers, ohne Werkzeug und in einem einzigen Fixierungsschritt. Sie ermöglicht zudem eine zuverlässige Fixierung auch bei Vorliegen geringfügiger Bauteiltoleranzen und vermeidet Handhabungsfehler bei der Montage.

In einer weiteren Ausführung ist der den wesentlichen Teil des Antennenaufbaus bildende langgestreckte Leiter bandförmig, und zwar speziell mit einem Verhältnis zwischen Breite und Höhe des bandförmigen Leiters 4:1 oder mehr, insbesondere mehr als 8:1. Mit dieser Ausführung lässt sich die Antenne in technologisch besonders einfacher Weise als Stanzbiegeteil aus einem geeigneten elektrisch leitenden Metallblech oder einer elektrisch leitenden Metallfolie herstellen und hat eine, auf den Materialeinsatz bezogen, große Abstrahlfläche.

Neben der Ausführung als Stanzbiegeteil sind auch mannigfache andere Ausführungen der erfindungsgemäßen Antenne möglich, etwa als 3D-gedrucktes Sinterteil oder Metal-Injection-Mould (MIM)-Teil oder auch als durch ein Erosionsverfahren aus einem geeigneten elektrisch leitenden Metallblech oder einer elektrisch leitenden Metallfolie gebildetem Teil.

Zur Erreichung einer elektromagnetisch vorteilhaften Leiter-Gesamtlänge bei zugleich einfacher Herstellbarkeit und Montage ist die Sende-/Empfangsantenne in einer weiteren vorteilhaften Ausführung im größten Teil ihrer Erstreckung mit mäanderförmigem Verlauf in einer Ebene auf der Oberseite und/oder an einer Seitenfläche des Headers angeordnet. In einer Ausgestaltung dieser Ausführung sind mindestens in einem Teilabschnitt des mäanderförmigen Verlaufs Versteifungsbrücken in den Mäander-Bögen vorgesehen.

In einer weiteren vorteilhaften Ausführung ist der oder ein federelastischer Abschnitt als Ω-förmige Klammer konfiguriert, die eine Anschlussbuchse im Header umgreift. Diese Ausführung eignet sich besonders für zahlreiche Geräte mit praktisch bewährten Header-Konstruktionen, die in ihrem Aufbau weitgehend genormte Anschlussbuchsen für Elektroden- und/oder Sensorleitungen umfassen. Für abweichend gestaltete Header kann die Antennenkonstruktion vorteilhaft durch entsprechend angepasste Formgebung eines funktional entsprechenden Klammerabschnittes modifiziert sein, oder es können elastische Haken o.ä. vorgesehen sein.

Mit der erfindungsgemäßen Antenne lassen sich, jedenfalls in Ausführungsformen der Erfindung, insbesondere einer oder mehrere der nachfolgend genannten Vorteile erreichen:
- Die Antenne ermöglicht eine automatisierte Herstellung des kompletten Headers und trägt somit zu Effizienzsteigerungen und Kostensenkungen bei der Geräteproduktion bei.
- Durch eine im Hinblick auf den Materialeinsatz und die mechanischen Eigenschaften optimierte Leiterlänge und Abstrahlfläche sind die Antenneneigenschaften optimiert.
- Durch den Formschluss und die optionale abschnittsweise Elastizität der Antenne bezüglich der Headerkomponenten-Kontur werden ungewollte und leistungsbeeinträchtigende Verformungen der Antenne bei der Montage weitgehend verhindert und somit aufwändige Nacharbeiten und Ausschuss weitgehend vermieden.

Vorteile und Zweckmäßigkeiten der Erfindung ergeben sich im Übrigen aus der nachfolgenden Beschreibung eines Ausführungsbeispiels anhand der Figuren. Von diesen zeigen:
- Fig. 1: eine perspektivische Ansicht einer herkömmlichen Sende-/Empfangsantenne im Header eines Einkammer-Herzschrittmachers,
- Fig. 2: eine perspektivische Gesamtansicht eines Einkammer-Herzschrittmachers mit darin eingezeichneter Ausführungsform der Sende-/Emp fangsantenne,
- Fig. 3: eine perspektivische Ausschnittsdarstellung des Header-Aufbaus mit der Sende-/Empfangsantenne des Schrittmachers nach Fig. 2, und
- Fig. 4A - 4D: skizzenartige perspektivische Darstellungen weiterer Ausführungsformen der erfindungsgemäßen Sende-/Empfangsantenne.

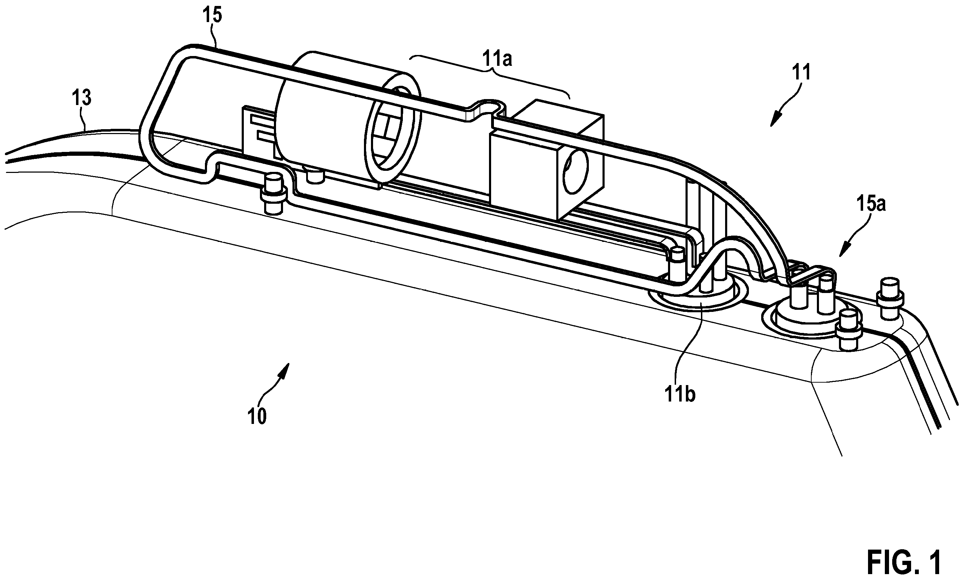

Fig. 1 zeigt in einer Ausschnittdarstellung einen Header 11 eines Herzschrittmachers 10 mit Anschlussbuchsen-Komponenten 11a und einem zugehörigen Anschlusselement 11b zur Verbindung mit (nicht dargestellten) elektronischen Komponenten in einem Gehäuse 13 des Schrittmachers. Weiterhin zeigt die Figur eine rahmenartige Sende-/Empfangsantenne 15 und ein dieser zugeordnetes Anschlusselement 15a. Es ist zu erkennen, dass die rahmenartige Sende-/Empfangsantenne 15 im Wesentlichen frei "schwebend" im Header 11 angeordnet und mechanisch nur am Anschlusselement 15a fixiert ist, welches natürlich außerdem ihrem elektrischen Anschluss dient.

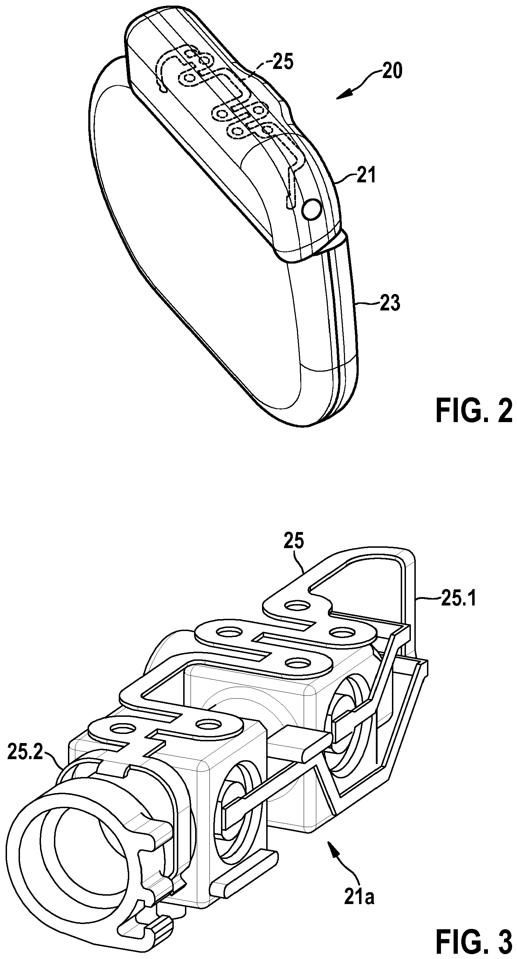

Fig. 2 zeigt eine Außenansicht eines erfindungsgemäß ausgestalteten Schrittmachers 20 mit einem Header 21 und einem Gehäuse 23, bei dem im Header 21 eine erfindungsgemäße Sende-/Empfangsantenne 25 angeordnet ist. Diese Sende-/Empfangsantenne 25 ist in Fig. 3 zusammen mit einem Buchsenanschlussblock 21a in perspektivischer Darstellung genauer gezeigt.

Es ist zu erkennen, dass die Antenne 25 über den größten Teil ihrer Erstreckung mit mäanderförmigem Verlauf in einer Ebene auf dem Buchsenanschlussblock 21a liegt und lediglich ihre beiden Endabschnitte 25.1 und 25.2 außerhalb dieser Ebene liegen. Der Endabschnitt 25.1 führt zum (nicht dargestellten) Anschlusselement zur Verbindung mit einer zugehörigen Telemetrie-Baugruppe im Schrittmachergehäuse, und Endabschnitt 25.2 ist als Ω-förmige Klammer gestaltet, die einen zylindrischen Abschnitt des Buchsenanschlussblocks 21a elastisch federnd umgreift. Es ist auch zu erkennen, dass innerhalb der Mäander des Antennenverlaufes brückenartige Verbindungen vorgesehen sind, die eine Erhöhung der Biegesteifigkeit des Antennenaufbaus bewirken. Weiterhin ist zu erkennen, dass die Sende-/Empfangsantenne 25 als flaches Stanzbiege- oder Formteil ausgebildet ist.

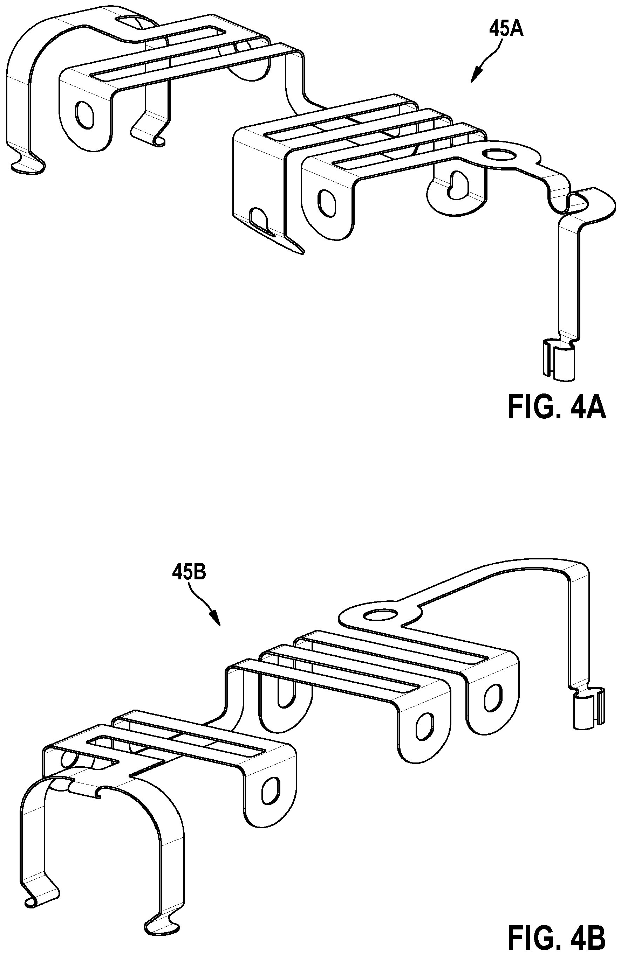

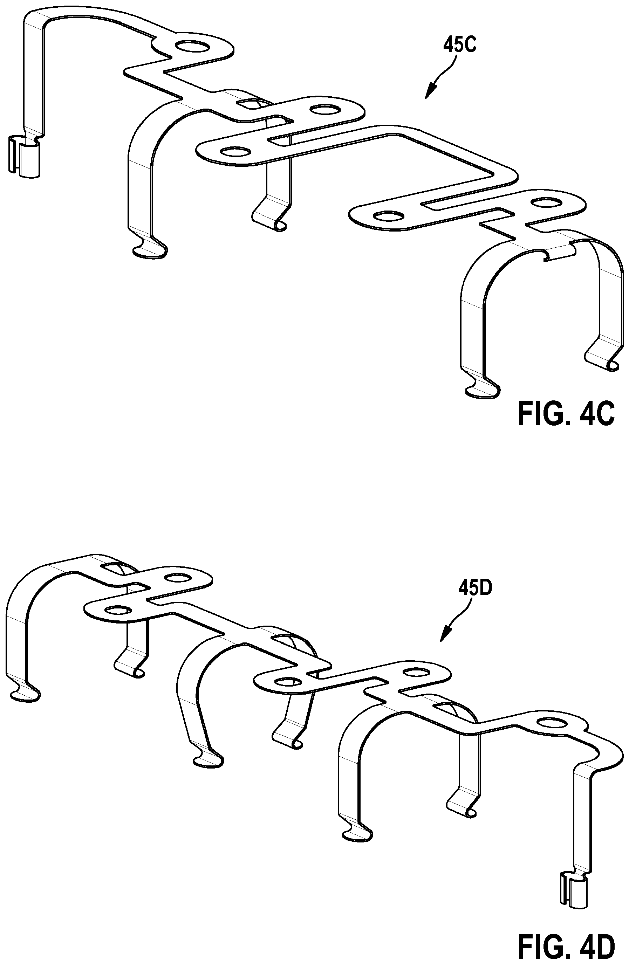

Die Figuren 4A bis 4D zeigen weitere Ausführungsformen von Sende-/Empfangsantennen 45A - 45D. Bei den Antennen 45A nach Fig. 4A und 45B nach Fig. 4B sind jeweils die Endabschnitte der Mäander des Antennen-Mittenabschnittes rechtwinklig abgekantet, womit ein zusätzlicher Formschluss mit einer im montierten Zustand darunter liegenden (nicht dargestellten) Buchsenbaugruppe des Headers gebildet werden kann. Die Sende-/Empfangsantennen 45C nach Fig. 4C und 45D nach Fig. 4D haben jeweils mehr als einen Klammerabschnitt und fixieren somit im montierten Zustand die jeweilige Antenne an mehreren Stellen durch elastisch federndes Umgreifen eines zylindrischen Abschnitts der jeweiligen, unter der Antenne liegenden (wiederum nicht dargestellten) Header-Baugruppe. Es ist darauf hinzuweisen, dass die Klammerabschnitte in allen Ausführungen neben ihrer mechanischen Fixierungsfunktion auch eine elektrische Funktion haben und die Antennencharakteristik vorteilhaft beeinflussen.

Im Übrigen ist die Ausführung der Erfindung auch in einer Vielzahl von Abwandlungen der hier gezeigten Beispiele und weiter oben hervorgehobenen Aspekte der Erfindung möglich.

## Patentansprüche

1. Implantierbares medizinelektronisches Gerät (20), welches einen Gerätekörper (23) und einen hierauf aufgesetzten Header (21) aufweist, mit einer Telemetrie-Baugruppe zur Signalübertragung nach und/oder von außerhalb des Körpers eines Patienten im implantierten Zustand des Gerätes, insbesondere zur drahtlosen bidirektionalen Kommunikation, wobei der Telemetrie-Baugruppe mindestens größtenteils eine aus einem langgestreckten Leiter räumlich geformte, im Bereich des Headers angeordnete Sende-/Empfangsantenne (25; 45A - 45D) zugeordnet ist, welche derart konfiguriert ist, dass sie einen Formschluss mit der Außenkontur von mindestens einem Teil (21a) des Headers hat und hierdurch im Header fixiert ist.

2. Gerät nach Anspruch 1, wobei wenigstens ein Abschnitt (25.2) des räumlich geformten langgestreckten Leiters federelastisch ausgeführt ist, derart, dass die Sende-/Empfangsantenne (25; 45A - 45D) durch elastischen Formschluss im Header (21) fixiert ist.

3. Gerät nach Anspruch 1 oder 2, wobei der langgestreckte Leiter bandförmig ist.

4. Gerät nach Anspruch 3, wobei das Verhältnis zwischen Breite und Höhe des bandförmigen Leiters 4:1 oder mehr, insbesondere mehr als 8:1, beträgt.

5. Gerät nach einem der vorangehenden Ansprüche, wobei die Sende-/Empfangsantenne (25; 45A - 45D) als Stanzbiegeteil, 3D-gedrucktes Sinterteil oder Metal-Injection-Mould-Teil ausgeführt ist.

6. Gerät nach einem der vorangehenden Ansprüche, wobei die Sende-/Empfangsantenne im größten Teil ihrer Erstreckung mit mäanderförmigem Verlauf in einer Ebene auf der Oberseite oder an einer Seitenfläche des Headers angeordnet ist.

7. Gerät nach Anspruch 6, wobei mindestens in einem Teilabschnitt des mäanderförmigen Verlaufs Versteifungsbrücken in den Mäander-Bögen vorgesehen sind.

8. Gerät nach einem der Ansprüche 2 bis 7, wobei der oder ein federelastischer Abschnitt (25.2) als Ω-förmige Klammer konfiguriert ist, die eine Anschlussbuchse (21a) im Header (21) umgreift.

9. Sende-/Empfangsantenne (25; 45A - 45D) einer Telemetrie-Baugruppe eines implantierbaren medizinelektronischen Gerätes (20), welches einen Gerätekörper (23) und einen hierauf aufgesetzten Header (21) aufweist, wobei die Sende-/Empfangsantenne aus einem langgestreckten Leiter räumlich geformt und derart konfiguriert ist, dass sie einen Formschluss mit der Außenkontur von mindestens einem Teil (21a) des Headers (21) bildet und hierdurch im Header fixierbar ist.

10. Sende-/Empfangsantenne nach Anspruch 9, wobei wenigstens ein Abschnitt (25.2) des räumlich geformten langgestreckten Leiters federelastisch ausgeführt ist, derart, dass die Sende-/Empfangsantenne (25; 45A - 45D) durch elastischen Formschluss im Header (21) fixierbar ist.

11. Sende-/Empfangsantenne nach Anspruch 9 oder 10, wobei der langgestreckte Leiter bandförmig ist.

12. Sende-/Empfangsantenne nach Anspruch 11, wobei das Verhältnis zwischen Breite und Höhe des bandförmigen Leiters 4:1 oder mehr, insbesondere mehr als 8:1, beträgt.

13. Sende-/Empfangsantenne nach einem der Ansprüche 9 bis 12, ausgeführt als Stanzbiegeteil, 3D-gedrucktes Sinterteil oder Metal-Injection-Mould-Teil (25; 45A - 45D).

14. Sende-/Empfangsantenne nach einem der Ansprüche 9 bis 13, welche im größten Teil ihrer Erstreckung einen mäanderförmigen Verlauf in einer Ebene hat.

15. Sende-/Empfangsantenne nach Anspruch 14, wobei mindestens in einem Teilabschnitt des mäanderförmigen Verlaufs Versteifungsbrücken in den Mäander-Bögen vorgesehen sind.

16. Sende-/Empfangsantenne nach einem der Ansprüche 10 bis 15, wobei der oder ein federelastischer Abschnitt (25.2) als Ω-förmige Klammer dazu konfiguriert ist, eine Anschlussbuchse (21a) im Header (21) zu umgreifen.
